(19) Europäisches Patentamt
European Patent Office
Office européen des brevets

(11) EP 0 997 451 B1

(12) EUROPÄISCHE PATENTSCHRIFT

(45) Veröffentlichungstag und Bekanntmachung des Hinweises auf die Patenterteilung:
**18.02.2004 Patentblatt 2004/08**

(51) Int Cl.[7]: **C07C 31/30**, C07C 29/70

(21) Anmeldenummer: **99118739.4**

(22) Anmeldetag: **22.09.1999**

(54) **Magnesiumethoxid mit hohem Grobkornanteil, Verfahren zu seiner Herstellung und seine Verwendung**

Magnesium ethoxide with a high proportion of coarse grains, method for its preparation and its use

Ethoxyde de magnésium à proportion élevée de gros grains, procédé de sa préparation et son utilisation

(84) Benannte Vertragsstaaten:
**AT BE CH CY DE DK ES FI FR GB GR IE IT LI LU MC NL PT SE**

(30) Priorität: **27.10.1998 DE 19849353**

(43) Veröffentlichungstag der Anmeldung:
**03.05.2000 Patentblatt 2000/18**

(73) Patentinhaber: **Degussa AG**
**40474 Düsseldorf (DE)**

(72) Erfinder:
• **Schork, Reinhold, Dr.**
**79618 Rheinfelden (DE)**
• **Standke, Burkhard, Dr.**
**79540 Lörrach (DE)**
• **Rauleder, Hartwig, Dr.**
**79618 Rheinfelden (DE)**
• **Frings, Albert-Johannes, Dr.**
**79618 Rheinfelden (DE)**
• **Horn, Michael, Dr.**
**79618 Rheinfelden (DE)**
• **Jenkner, Peter, Dr.**
**79618 Rheinfelden (DE)**
• **Monkiewicz, Jaroslaw, Dr.**
**79618 Rheinfelden (DE)**

(56) Entgegenhaltungen:
DE-A- 3 412 337        FR-A- 2 210 596
US-A- 3 657 361

• **DATABASE WPI Section Ch, Week 199621 Derwent Publications Ltd., London, GB; Class A17, AN 1996-205468 XP002134773 & JP 08 073388 A (NIPPON SODA CO), 19. März 1996 (1996-03-19)**
• **PATENT ABSTRACTS OF JAPAN vol. 011, no. 115 (C-415), 10. April 1987 (1987-04-10) & JP 61 260086 A (IDEMITSU PETROCHEM CO LTD), 18. November 1986 (1986-11-18)**

**Beschreibung**

Die vorliegende Erfindung betrifft partikelförmiges Magnesiumethylat mit Grobkornanteil, ein Verfahren zu seiner Herstellung und seine Verwendung.

Verfahren zur Herstellung von Magnesiumethylat sind seit geraumer Zeit bekannt. Eine technische Herstellroute ist die direkte Synthese aus metallischem Magnesium und Ethanol. Wie schon beispielsweise H. D. Lutz in der Zeitschrift für anorganische und allgemeine Chemie, Band 356, 1968, Seite 132 ff., beschreibt, benötigt man zum Starten der Reaktion einen Katalysator. Meist wird als Katalysator Jod eingesetzt. Dies hat den Nachteil, dass das so hergestellte Magnesiumethylat Spuren des Startkatalysators enthält, was sich negativ auf die Anwendungseigenschaften auswirken kann, beispielsweise bei der Verwendung als Katalysatorprecursor für Ziegler-Katalysatoren oder für die Buchkonservierung. Verzichtet man auf den Einsatz von Katalysatoren, zeigt die Erfahrung, dass die Reaktion zwischen Ethanol und Magnesium nicht sicher anspringt. Gerade für technische Prozesse birgt ein unkontrollierbares Anspringverhalten hohe Risiken. Bei den bekannten Verfahren zur Herstellung von Magnesiumethylat sind auch die langen Reaktionszeiten wirtschaftlich wenig vorteilhaft. Zur vollständigen Reaktion von Ethanol mit Magnesium sind Reaktionszeiten von mehr als 24 Stunden erforderlich. Bricht man die Reaktion vorzeitig ab, so bleibt nicht abreagiertes metallisches Magnesium im Gemisch mit Magnesiumethylat zurück. Eine Trennung ist mit vertretbarem technischen Aufwand nicht durchführbar. Ferner verschlechtert metallisches Magnesium die Anwendungseigenschaften von Magnesiumethylat. Darüber hinaus ist bei der Magnesiumethylatsynthese nach bekannten Verfahren festzustellen, dass stets ein Magnesiumethylatkorn mit erheblichen Anteilen an unerwünschtem Unterkorn entsteht. Dieser Feinkorn- bzw. Staubanteil verschlechtert ebenfalls die Anwendungseigenschaften des Produkts. Dieser Produktanteil wird bei der Herstellung von Magnesiumethoxid aufwendig entfernt, beispielsweise durch Sieben oder Sichten, und verworfen.

Der vorliegenden Erfindung lag daher die Aufgabe zugrunde, ein Verfahren bereitzustellen, welches die Herstellung von Magnesiumethoxid in besonders wirtschaftlicher Weise ermöglicht.

Die gestellte Aufgabe wird erfindungsgemäß entsprechend den Angaben der Patentansprüche gelöst.

Überraschenderweise wurde nun gefunden, dass partikelförmiges Magnesiumethoxid mit hohem Grobkomanteil in einfacher und wirtschaftlicher Weise erhältlich ist, wenn man reines metallisches, gegebenenfalls aktiviertes Magnesium mit reinem, wasserfreiem Ethanol in flüssiger Phase unter Druck bei einer Temperatur oberhalb der Siedetemperatur von Ethanol bei Normaldruck (78 °C) in Kontakt bringt, abreagieren lässt und das Produkt gewinnt. Die Reaktion kann so in einfacher und wirtschaftlicher Weise sicher und ohne einen das Produkt verunreinigenden Katalysator gestartet werden. Ferner kann durch das vorliegende Verfahren ein Verbleib von metallischem, nicht abreagiertem Magnesium vollständig vermieden werden. Die Reaktionszeiten des vorliegenden Verfahrens liegen in der Regel deutlich unter 24 Stunden, wodurch eine besonders wirtschaftliche Betriebsweise ermöglicht wird. Weiterhin resultiert aus dem vorliegenden Verfahren ein Produkt mit einem vergleichsweise geringen Anteil an unerwünschtem Unterkorn bzw. Staubanteil.

Gegenstand der vorliegenden Erfindung ist somit ein partikelförmiges Magnesiumethoxid mit Grobkornanteil, das durch Umsetzen von metallischem, gegebenenfalls aktivierten Magnesium mit flüssigem Ethanol unter Druck bei einer Temperatur oberhalb 78 °C und anschließender Produktgewinnung erhältlich ist.

Bei der erfindungsgemäßen Umsetzung bringt man vorzugsweise flüssiges Ethanol und festes, metallisches Magnesium in Kontakt und erhitzt unter Druck auf eine Temperatur oberhalb 78 °C in einer Weise, sodass flüssiges Ethanol als Reaktionspartner zur Verfügung steht und gebildeter Wasserstoff aus dem Reaktionsraum ausgeschleust wird.

In der Regel setzt man für die Durchführung der erfindungsgemäßen Umsetzung getrocknetes Ethanol im Überschuss ein, sodass Ethanol gleichzeitig als Lösemittel dienen kann.

Das bei der erfindungsgemäßen Umsetzung anfallende Magnesiumethoxid gewinnt man anschließend geeigneterweise durch Filtration oder durch Einengen des Produktgemisches bei einer Temperatur im Bereich von 0 bis 180 °C unter vermindertem Druck. Vor der Produktgewinnung kann man die erhaltene Produktmischung kühlen, insbesondere auf eine Temperatur von 20 bis 78 °C.

Sowohl die erfindungsgemäße Umsetzung als auch die Gewinnung des erfindungsgemäßen Magnesiumethoxids führt man bevorzugt unter Schutzgas durch. Insbesondere setzt man ein in an sich bekannter Weise getrocknetes sowie $CO_2$-freies Schutzgas ein, beispielsweise Stickstoff.

Gegenstand der vorliegenden Erfindung ist ebenfalls ein Verfahren zur Herstellung von partikelförmigem Magnesiumethoxid mit Grobkomanteil, wobei man metallisches, gegebenenfalls aktiviertes Magnesium mit Ethanol in flüssiger Phase umsetzt und nachfolgend das Magnesiumethoxid gewinnt, das dadurch gekennzeichnet ist, dass man die Umsetzung unter Druck oberhalb 1 bar abs. bei einer Temperatur oberhalb 78 °C durchführt.

Vorzugsweise stellt man beim erfindungsgemäßen Verfahren die Reaktionstemperatur auf einen Wert von > 78 bis 200 °C, besonders vorzugsweise von 80 bis 130°C, ganz besonders vorzugsweise von 100 bis 130 °C, und den Druck im Reaktionsraum auf einen Wert oberhalb 1 bar abs., besonders vorzugsweise auf einen Wert von 2 bis 6 bar abs., ein.

Das beim erfindungsgemäßen Verfahren eingesetzte metallische Magnesium besitzt bevorzugt eine Körnung von 20 bis 5.000 µm. Bevorzugt wird es als reines Magnesiummetall eingesetzt. Man kann es aber auch vor der Umsetzung aktivieren, beispielsweise durch Anätzen.

Zum Starten der Reaktion kann man dem Reaktionsgemisch aber auch einen Katalysator zusetzen. Geeigneterweise setzt man als Katalysator eine anorganische oder organische Halogenverbindung, vorzugsweise ein Quecksilberhalogenid, eine anorganische oder organische Säure, wie HCl oder Essigsäure, oder ein Alkylorthoformiat, wie Tetraethylorthoformiat (TEOF), ein.

Insbesondere startet man beim erfindungsgemäßen Verfahren die Reaktion durch eine kurzzeitige Temperaturerhöhung auf eine Temperatur oberhalb 78 °C, bevorzugt > 90 °C. Geeigneterweise führt man im weiteren Verlauf der Umsetzung das vorliegende Verfahren so durch, dass die Spitze der Wasserstoffentwicklung bei einer Temperatur unterhalb 90 °C abläuft, der entstehende Wasserstoff aus dem Reaktionsraum abgeführt wird und die restliche Umsetzung bei einer Temperatur oberhalb 90 °C erfolgt, ebenfalls unter Ausschleusung des gebildeten Wasserstoffs. Beim erfindungsgemäßen Verfahren ist die Umsetzung im allgemeinen nach 16 Stunden quantitativ abgelaufen, sodass in vorteilhafter Weise praktisch keine Magnesiummetallreste im Produktgemisch verbleiben. Das Produkt wird im allgemeinen in oben beschriebener Weise aus dem Produktgemisch gewonnen.

Erfindungsgemäß hergestelltes bzw. erhältliches partikelförmiges Magnesiumethoxid besitzt vorteilhafterweise einen hohen Anteil an Grobkorn. Die Bestimmung des Komanteils erfolgt in der Regel unter trockenem Schutzgas durch Siebanalyse.

Gegenstand der vorliegenden Erfindung ist daher auch ein partikelförmiges Magnesiumethoxid mit Grobkornanteil, das durch Siebkornfraktionen ≤ 500 µm (Maschenweite) mit einem Anteil von < 40 Gew.-% und > 500 µm mit einem Anteil von ≥60 Gew.-% gekennzeichnet ist.

Vorzugsweise ist erfindungsgemäßes Magnesiumethoxid durch eine Siebfraktion > 500 µm (Maschenweite) mit einem Anteil von ≥80 Gew.-% gekennzeichnet.

Insbesondere beträgt beim erfindungsgemäßen Magnesiumethoxid der Anteil einer Siebkornfraktion > 800 µm (Maschenweite) mehr als 40 Gew.-%, bezogen auf die Gesamtmenge.

Ferner ist erfindungsgemäßes Magnesiumethoxid bevorzugt durch eine Siebfraktion < 315 µm (Maschenweite) mit einem Anteil kleiner 10 Gew.-%, besonders vorzugsweise von 0,01 bis 5 Gew.-%, gekennzeichnet D. h., erfindungsgemäßes partikelförmiges Magnesiumethoxid besitzt in hervorragender und besonders wirtschaftlicher Weise einen hohen Anteil an Grobkorn und in besonders überraschender und vorteilhafter Weise nur einen vergleichsweise geringen Anteil an Unterkorn, wobei der Anteil an Feinstaub geeigneterweise fast gegen Null geht.

Im allgemeinen führt man das erfindungsgemäße Verfahren wie folgt aus: In einem beheizbaren Druckreaktor aus Edelstahl, ausgerüstet mit regelbarem Überdruckventil, kann man in einer trockenen Schutzgasatmosphäre, beispielsweise Stickstoff, wasserfreies Ethanol und partikelförmiges metallisches Magnesium vorgelegen und auf eine Temperatur oberhalb 78 °C aufheizen. Durch den Dampfdruck des verwendeten Ethanols und die Wasserstoffentwicklung gemäß Reaktion I kann sich im geschlossenen Reaktor ein Druck oberhalb des Umgebungsdrucks entwickeln.

$$\text{Reaktion I: } Mg + 2C_2H_5OH \rightarrow Mg(OC_2H_5)_2 + H_2$$

Überschreitet dieser die am Überdruckventil eingestellte Grenze, kann das Ventil öffnen und es entweicht Wasserstoff und Ethanoldampf. Das entspannte Gasgemisch wird in der Regel durch einen Kühler geleitet wobei Ethanol kondensiert, während Wasserstoff gasförmig abgeführt wird. Über eine Dosierpumpe, welche den im Reaktor herrschenden Überdruck überwinden kann, kann man das kondensierte Ethanol in den Reaktor zurückführen. Diese Reaktionsführung hat insbesondere den Vorteil, dass kein Überdruck geeignetes Kondensationssystem verwendet werden muss. Steht ein überdrucksicheres Kondensationssystem zur Verfügung, kann das Überdruckregelventil ebenso am Kopf des Kondensationssystems angebracht werden, in einer Weise, dass das entspannte Gas nach dem Druckregelventil hauptsächlich Wasserstoff enthält. Der Rückfluss des Kondensationssystems, Ethanol, wird im allgemeinen in den Reaktor zurückgeführt, sodass auf eine Rückführung von Ethanol unter Drucküberwindung verzichtet werden kann.

Die Reaktion kann auch drucklos gestartet werden, z. B. unter Verwendung von literaturbekannten Katalysatoren, wie Halogene, Halogenverbindungen, Säuren, Quecksilberverbindungen oder Alkylorthoformiate, und anschließend unter Druckerhöhung weitergeführt werden. Dies hat im allgemeinen den Vorteil, dass Druckspitzen, hervorgerufen durch die anfangs sehr heftige Reaktion bei erhöhter Temperatur (>78 °C) gemildert werden können. Ein nicht beherrschbarer Druckanstieg im Reaktor stellt ein erfebliches Gefahrenpotential dar.

Eine weitere bevorzugte Methode der Reaktionsführung besteht darin, die Reaktion unter Verzicht von Katalysator bei Normaldruck und Siedetemperatur von Ethanol (78 °C) zu beginnen und im Zuge des Reaktionsfortschritts Druck und Temperatur in der Anlage zu erhöhen. Druckspitzen können auf diese Weise ebenfalls vermieden werden. Sollten Probleme bezüglich Anspringen der Reaktion bestehen, kann man durch kurzzeitige Temperatur- und Druckerhöhung die Reaktion starten. Eine unkontrollierte Wasserstoffentwicklung kann man dadurch abfangen, dass sofort nach Start der Reaktion Druck und Temperatur im Reaktor durch eine Entspannung über das Druckregelventil abgesenkt werden.

Die Produktgewinnung kann beim erfindungsgemäßen Verfahren in bereits oben genannter Weise durchgeführt werden

Das erfindungsgemäße Verfahren besitzt ferner die Vorteile, dass insbesondere bei Verwendung von partikelförmigem Magnesium mit einer mittleren Partikelgröße > 200 μm , vorzugsweise > 800 μm, die Reaktionszeit weniger als 24 Stunden beträgt, kein metallisches Magnesium im Produkt verbleibt und deutlich weniger Unterkorn entsteht als bei der Herstellung des Produkts bei Normaldruck nach herkömmlichen Verfahren.

Erfindungsgemäßes, partikelförmiges Magnesiumethoxid mit Grobkornanteil findet aufgrund seiner vorteilhaften Eigenschaften insbesondere, aber nicht ausschließlich, Verwendung als Precursor für Ziegler-Natta-Katalysatoren, als Precursor für Keramik sowie als Precursor für Buchkonservierungsmittel.

Durch die nachfolgenden Beispiele wird die vorliegende Erfindung näher erläutert:

**Beispiele**

Reaktionsapparatur:

1-I-Edelstahlautoklav mit wandgängigem Rührer, Druckmesseinrichtung (Dosenmanometer), Temperaturmesseinrichtung (Pt 100 Messfühler, Temperierung über Thermostat, Flüssigkeitsdosierpumpe (Membranpumpe Prominent Gamma/4-/, regelbares Überdruckventil, Kondensationssystem (Intensivkühler aus Glas), Gasuhr (Trommelgaszähler TG3, Fa. Ritter) und Schutzgasüberlagerung (Stickstoff).

Trocknungsapparatur:

Schutzgas (Stickstoff) überlagerter Labor-Rotationsverdampfer (2-I-Verdampferkolben aus Glas) mit Vakuumeinrichtung.

Durchführung der Reaktion:

24,3 g partikelförmiges, metallisches Magnesium (d50 > 800 μm, Reinheit > 99 %) werden unter Schutzgasbeschleierung zusammen mit 356 g Ethanol im Edelstahlautoklaven vorgelegt. Nach Verschließen der Apparatur wird das Überdruckventil auf den gewünschten Reaktorinnendruck eingestellt und die Mischung auf Reaktionstemperatur gebracht. Bei Erreichen der vorgewählten Temperatur und des vorgewählten Drucks entweicht Wasserstoff und Ethanoldampf über das Druckregelventil in das Kondensationssystem. Auskondensiertes Ethanol wird in der Menge über die Membrandosierpumpe in den Reaktor zurückgepumpt wie Kondensat am Kühler gebildet wird. Der entstehende Wasserstoff wird über den Trommelgaszähler aus der Reaktionsapparatur abgeführt. Der Wasserstoff-Volumenstrorn wird gemessen. Lässt sich keine Wasserstoffentwicklung mehr feststellen, wird die Reaktion beendet, indem der Reaktor entspannt und abgekühlt wird. Die Suspension (Magnesiumethylat in Ethanol) wird in den 2-I-Kolben des Rotationsverdampfers überführt und dort bei 100 °C und unter vermindertem Druck (bis 1 mbar) innerhalb von 2,5 Stunden getrocknet. Anschließend wird das Produkt einer Korngrößenanalyse unterzogen (Siebanalyse). Zusätzlich wird der Gehalt an nicht abreagiertem, metallischem Magnesium indirekt über die Menge an gebildetem Wasserstoff bei Versetzen des Produkts mit wässriger Chlorwasserstoffsäure bestimmt.

**1. (NP6994) Herstellung von Magnesiumethylat aus Ethanol und Magnesium bei 110 °C und einem Druck von 3 bar**

Es wird wie unter "Durchführung der Reaktion" beschrieben verfahren. Die Temperatur des Heizmediums (Siliconöl) des Thermostaten beträgt 170 °C. Bei einem Druck von 3 bar im Reaktor beträgt die Temperatur im Reaktor 110 °C. Ca. 5 Minuten nach Erreichen der Reaktionstemperatur wird die maximale Wasserstoffentwicklung von ca. 30 l/h gemessen. Nach ca. 6 Stunden ist die Reaktion beendet, es findet keine Wasserstoffentwicklung mehr statt. Das Produkt wird wie oben beschrieben getrocknet und analysiert.

| Siebanalyse | |
|---|---|
| >800 µm | 55,7 Gew.-% |
| 500 - 800 µm | 36,4 Gew.-% |
| 315 - 500 µm | 7,1 Gew.-% |
| 200 - 315 µm | 0,6 Gew.-% |
| 100 - 200 µm | 0 Gew.-% |
| <100 µm | 0,1 Gew.-% |
| Metallisches Magnesium | <0,02 Gew.-% |

**2. (NP6694) Herstellung von Magnesiumethylat aus Ethanol und Magnesium bei 120 °C und einem Druck von 4 bar**

Es wird wie unter "Durchführung der Reaktion" beschrieben verfahren. Die Temperatur des Heizmediums (Siliconöl) des Thermostaten beträgt 170°C.

Bei einem Druck von 4 bar im Reaktor beträgt die Temperatur im Reaktor 120 °C. Ca. 5 Minuten. Nach Erreichen der Reaktionstemperatur wird die maximale Wasserstoffentwicklung von ca. 35 l/h gemessen. Nach ca. 3,5 Stunden ist die Reaktion beendet, es findet keine Wasserstoffentwicklung mehr statt. Das Produkt wird wie oben beschrieben getrocknet und analysiert.

| Siebanalyse | |
|---|---|
| >800 µm | 46,2 Gew.-% |
| 500 - 800 µm | 36,4 Gew.-% |
| 315 - 500 µm | 13,1 Gew.-% |
| 200 - 315 µm | 3,3 Gew.-% |
| 100 - 200 µm | 0,8 Gew.-% |
| <100 µm | 0,1 Gew.-% |
| Metallisches Magnesium | <0,02 Gew.-% |

**3 Herstellung von Magnesiumethylat aus Ethanol und Magnesium bei 78 °C und Umgebungsdruck (1 bar, Vergleichsbeispiel, NP6894)**

Es wird wie unter Durchführung der Reaktion" beschrieben verfahren, jedoch mit dem Unterschied: Die verwendeten Magnesiumspäne werden mit 0,1 n wässriger HCl zur Entfernung der Oxidschicht gewaschen und unter Schutzgas bei 100 °C getrocknet, um die Reaktivität zu erhöhen, ohne diesen Schritt springt die Reaktion nicht sicher an. Die Temperatur des Heizmediums (Siliconöl) des Thermostaten beträgt 120 °C. Bei einem Druck von 1 bar im Reaktor (Umgebungsdruck) beträgt die Temperatur im Reaktor 78 °C. Ca. 5 Minuten nach Erreichen der Reaktionstemperatur wird die maximale Wasserstoffentwicklung von ca. 6 l/h gemessen. Nach ca. 7 Stunden wird dem Reaktor eine Probe entnommen. Das Reaktionsprodukt enthält noch erhebliche Mengen mit dem Auge sichtbares nicht abreagiertes Magnesium. Erst nach einer Reaktionszeit von mehr als 24 Stunden ist die Wasserstoffentwicklung beendet, und der Reaktor wird abgekühlt. Das Produkt wird wie oben beschrieben getrocknet und analysiert.

| Siebanalyse | |
|---|---|
| >800 µm | 5,9 Gew.-% |
| 500 - 800 µm | 39,5 Gew.-% |
| 315 - 500 µm | 42,9 Gew.-% |
| 200 - 315 µm | 8,6 Gew.-% |
| 100 - 200 µm | 2,6 Gew.-% |
| <100 µm | 0,5 Gew.-% |
| Metallisches Magnesium | <0,02 Gew.-% |

Die Partikelgrößenverteilung zeigt eine deutliche Zunahme unerwünschten Unterkorns (Anteil an Partikeln <315 µm ist >11 %).

**Patentansprüche**

1. Partikelförmiges Magnesiumethoxid mit Grobkornanteil **gekennzeichnet durch** Siebkomfraktionen
   ≤ 500 µm mit einem Anteil von < 40 Gew.-% und
   > 500 µm mit einem Anteil von ≥ 60 Gew.-%.

2. Partikelförmiges Magnesiumethoxid nach Anspruch 1
   **gekennzeichnet durch**
   eine Siebkornfraktion
   < 315 µm mit einem Anteil < 10 Gew.-%.

3. Partikelförmiges Magnesiumethoxid nach Anspruch 1 oder 2 **gekennzeichnet durch**
   eine Siebkornfraktion
   < 315 µm mit einem Anteil von 0,01 bis 5 Gew.-%.

4. Partikelförmiges Magnesiumethoxid nach mindestens einem der Ansprüche 1 bis 3
   **gekennzeichnet durch**
   eine Siebkornfraktion
   > 500 µm mit einem Anteil von ≥ 80 Gew.-%.

5. Partikelförmiges Magnesiumethoxid nach mindestens einem der Ansprüche 1 bis 4,
   **dadurch gekennzeichnet,**
   **dass** der Anteil einer Siebkornfraktion > 800 µm mehr als 40 Gew.-% bezogen auf die Gesamtmenge beträgt.

6. Partikelförmiges Magnesiumethoxid mit Grobkornanteil nach den Ansprüchen 1 bis 5 erhältlich durch Umsetzen von metallischem, gegebenenfalls aktiviertem Magnesium mit flüssigem Ethanol unter Druck oberhalb 1 bar abs. bei einer Temperatur oberhalb 78 °C und anschließender Produktgewinnung.

7. Verfahren zur Herstellung von partikelförmigem Magnesiumethoxid mit Grobkornanteil nach den Ansprüchen 1 bis 6, wobei man metallisches, gegebenenfalls aktiviertes Magnesium mit Ethanol in flüssiger Phase umsetzt und nachfolgend das Magnesiumethoxid gewinnt,

**dadurch gekennzeichnet,**
**dass** man die Umsetzung unter Druck oberhalb 1 bar abs. bei einer Temperatur oberhalb 78 °C durchführt.

8. Verfahren nach Anspruch 7,
   **dadurch gekennzeichnet,**
   **dass** man flüssiges Ethanol und festes, metallisches Magnesium in Kontakt bringt, unter Druck auf eine Temperatur oberhalb 78 °C in einer Weise erhitzt, sodass flüssiges Ethanol als Reaktionspartner zur Verfügung steht und gebildeter Wasserstoff aus dem Reaktionsraum ausgeschleust wird.

9. Verfahren nach Anspruch 7 oder 8,
   **dadurch gekennzeichnet,**
   **dass** man die Reaktionstemperatur auf einen Wert von >78 bis 200 °C und den Druck im Reaktionsraum auf einen Wert oberhalb 1 bar abs. einstellt.

10. Verfahren nach mindestens einem der Ansprüche 7 bis 9,
    **dadurch gekennzeichnet,**
    **dass** man metallisches Magnesium in einer Körnung von 20 bis 5.000 μm einsetzt.

11. Verfahren nach mindestens einem der Ansprüche 7 bis 10,
    **dadurch gekennzeichnet,**
    **dass** man die Reaktion durch eine kurzzeitige Temperaturerhöhung auf eine Temperatur oberhalb 90 °C startet.

12. Verfahren nach mindestens einem der Ansprüche 7 bis 11,
    **dadurch gekennzeichnet,**
    **dass** die Spitze der Wasserstoffentwicklung bei einer Temperatur unterhalb 90 °C abläuft, der entstehende Wasserstoff aus dem Reaktionsraum abgeführt wird und die restliche Reaktion bei einer Temperatur oberhalb 90 °C erfolgt, ebenfalls unter Ausschleusung des gebildeten Wasserstoffs.

13. Verwendung von partikelförmigem Magnesiumethoxid mit Grobkornanteil nach den Ansprüchen 1 bis 12 als Precursor für Ziegler-Natta-Katalysatoren, als Precursor für Keramik sowie als Precursor für Buchkonservierungsmittel.

**Claims**

1. Particulate magnesium ethoxide having a proportion of coarse grains
   **characterised by**
   sieve fractions of
   < 40 wt.% of grains ≤ 500 μm and
   ≥ 60 wt.% of grains > 500 μm.

2. Particulate magnesium ethoxide according to Claim 1,
   **characterised by**
   a sieve fraction of
   < 10 wt.% of grains < 315 μm.

3. Particulate magnesium ethoxide according to
   Claim 1 or 2,
   **characterised by**
   a sieve fraction of
   from 0.01 to 5 wt.% of grains < 315 μm.

4. Particulate magnesium ethoxide according to at least one of Claims 1 to 3,
   **characterised by**
   a sieve fraction of
   ≥ 80 wt.% of grains > 500 μm.

5. Particulate magnesium ethoxide according to at least one of Claims 1 to 4,

**characterised in that**
the proportion of a sieve fraction of grains > 800 µm is more than 40 wt.%, in relation to the total quantity.

6. Particulate magnesium ethoxide having a proportion of coarse grains according to Claims 1 to 5, which is obtainable by the reaction of metallic, optionally activated, magnesium with liquid ethanol at a pressure exceeding 1 bar absolute at a temperature exceeding 78°C, followed by recovery of the product.

7. Process for the preparation of particulate magnesium ethoxide having a proportion of coarse grains according to Claims 1 to 6, wherein metallic, optionally activated, magnesium is reacted in a liquid phase with ethanol and the magnesium ethoxide is subsequently recovered,
**characterised in that**
the reaction is carried out at a pressure exceeding 1 bar absolute at a temperature exceeding 78°C.

8. Process according to Claim 7,
**characterised in that**
liquid ethanol and solid metallic magnesium are contacted, heated at pressure to a temperature exceeding 78°C in a manner such that liquid ethanol is available as a reagent and hydrogen which is formed is transferred out of the reaction chamber.

9. Process according to Claim 7 or 8,
**characterised in that**
the reaction temperature is adjusted to a value of from > 78 to 200°C and the pressure in the reaction chamber is adjusted to a value exceeding 1 bar absolute.

10. Process according to at least one of Claims 7 to 9,
**characterised in that**
metallic magnesium having a grain size of from 20 to 5,000 µm is utilised.

11. Process according to at least one of Claims 7 to 10,
**characterised in that**
the reaction is initiated by a brief increase in temperature to a temperature exceeding 90°C.

12. Process according to at least one of Claims 7 to 11,
**characterised in that**
peak hydrogen evolution proceeds at a temperature below 90°C, the hydrogen which arises is removed from the reaction chamber, and the remainder of the reaction takes place at a temperature exceeding 90°C, likewise with the hydrogen which is formed being transferred out.

13. Use of particulate magnesium ethoxide having a proportion of coarse grains according to Claims 1 to 12 as a precursor for Ziegler-Natta catalysts, as a precursor for ceramics as well as a precursor for book conservation agents.

**Revendications**

1. Ethoxyde de magnésium particulaire contenant des gros grains,
**caractérisé par** des
fractions granulométriques
≤ 500 µm dans une proportion < 40 % en poids et
< 500 µm dans une proportion ≥ 60 % en poids.

2. Ethoxyde de magnésium particulaire selon la revendication 1,
**caractérisé par**
une fraction granulométrique
< 315 µm dans une proportion < 10 % en poids.

3. Ethoxyde de magnésium particulaire selon la revendication 1 ou 2,
**caractérisé par**

une fraction granulométrique
< 315 µm dans une proportion de 0,01 à 5 % en poids.

**4.** Ethoxyde de magnésium particulaire selon au moins l'une quelconque des revendications 1 à 3,
**caractérisé par**
une fraction granulométrique
> 500 µm dans une proportion ≥ 80 % en poids.

**5.** Ethoxyde de magnésium particulaire selon au moins l'une quelconque des revendications 1 à 4,
**caractérisé en ce que**
la proportion d'une fraction granulométrique > 800 µm est supérieure à 40 % en poids par rapport à la quantité totale.

**6.** Ethoxyde de magnésium particulaire contenant des gros grains selon les revendications 1 à 5, pouvant être obtenu par réaction de magnésium métallique, éventuellement activé, avec de l'éthanol liquide sous une pression supérieure à 1 bar absolu à une température supérieure à 78°C suivie de l'extraction du produit.

**7.** Procédé de fabrication d'éthoxyde de magnésium particulaire contenant des gros grains selon les revendications 1 à 6, dans lequel on fait réagir du magnésium métallique, éventuellement activé, avec de l'éthanol en phase liquide et on extrait ensuite l'éthoxyde de magnésium,
**caractérisé en ce qu'**
on réalise la réaction sous une pression supérieure à 1 bar absolu à une température supérieure à 78°C.

**8.** Procédé selon la revendication 7,
**caractérisé en ce que**
on met en contact de l'éthanol liquide et du magnésium métallique solide, on chauffe sous pression à une température supérieure à 78°C de manière à disposer d'éthanol liquide comme partenaire réactionnel et à évacuer l'hydrogène formé hors de la chambre de réaction.

**9.** Procédé selon la revendication 7 ou 8,
**caractérisé en ce que**
on ajuste la température de réaction sur une valeur allant de > 78 jusqu'à 200°C et la pression dans la chambre de réaction sur une valeur supérieure à 1 bar absolu.

**10.** Procédé selon au moins l'une quelconque des revendications 7 à 9,
**caractérisé en ce que**
on utilise du magnésium métallique d'une granulométrie comprise entre 20 et 5.000 µm.

**11.** Procédé selon au moins l'une quelconque des revendications 7 à 10,
**caractérisé en ce que**
on amorce la réaction en augmentant brièvement la température à une température supérieure à 90°C.

**12.** Procédé selon au moins l'une quelconque des revendication 7 à 11,
**caractérisé en ce que**
le pic du développement d'hydrogène se déroule à une température inférieure à 90°C, l'hydrogène qui se forme est évacué hors de la chambre de réaction et la réaction restante a lieu à une température supérieure à 90°C, également avec éclusage de l'hydrogène formé.

**13.** Utilisation d'éthoxyde de magnésium particulaire à gros grains, selon les revendications 1 à 12, en tant que précurseur de catalyseurs Ziegler-Natta, en tant que précurseur pour la céramique et en tant que précurseur pour des agents de conservation de livres.